# EUROPEAN PATENT APPLICATION

(11) **EP 2 228 056 A2**
(43) Date of publication of application: **15.09.2010**
(21) Application number: 10450026.9
(22) Date of filing: 22.02.2010
(51) Int. Cl.: A61K 9/00, A61K 31/445

(54) **Tolperisone controlled release tablet**

(30) Priority: 09.03.2009 US 158440
(71) Applicant: Sanochemia Pharmazeutika AG, 1091 Wien (AT)
(72) Inventor: Welzig, Stefan, 1030 Wien (AT); Rothenburger, Jan, 7064 Oslip (AT); Kälz, Beate, 7035 Steinbrunn (DE); Gungl, József, 9400 Sopron (HR); Gerdes, Klaus, 40233 Düsseldorf (DE)
(74) Representative: Dungler, Karin

(57) **Abstract**

The invention refers to a GRDDS (Gastro Retentive Drug Delivery System) containing tolperisone and having a 4-MMPPO fraction of less than 1.5 ppm for the extended release of the active substance tolperisone while avoiding the formation of 4-MMPPO in the gastrointestinal tract.

## Description

### 1. Subject Matter

The subject matter of the present invention relates to the production of a tolperisone extended release tablet (GRDDS, Gastro Retentive Drug Delivery System) for the controlled release of the active substance tolperisone, with the objective of reaching a constant active substance level in the blood while avoiding the risk of exposing the patient to the potential genotoxic impurity 2-methyl-1-(4-methylphenyl)-propenone (4-MMPPO).

### 2. Prior Art

### IR (instant release) tablets:

Commercially available IR tablets include, for example, Mydocalm and Mydeton. These tablets contain 4-MMPPO in significant quantities.

### CR (controlled release) tablets:

Currently, no CR formulations are commercially available.

### 2. Inventive Step

Problem: Tolperisone tablets are unable to ensure a constant concentration of the active substance (tolperisone) in the blood. However, especially in cases of spastic muscle cramps, a constant efficacy, in particular throughout the night, is very important to the quality of life of the patients. Known tablet formulations release the active substance tolperisone in the intestine at pH 4 to 7. In this pH range, tolperisone breaks down into 4-MMPPO and piperidine, which can be demonstrated in laboratory tests. Thus, the patient is exposed to an uncontrollable quantity of 4-MMPPO.

Solution: Proposed are floating tolperisone tablets with the controlled release of the active substance tolperisone in the stomach at pH 1 to 2.

A CR floating tablet with tolperisone as the active substance, which can also be prepared free from 4-MMPPO, is currently not known.

### 4. Description of the invention:

### 4.1 Principle:

### Brief explanation and principle of the floatable oral therapeutic system

For a number of reasons, it may be medically important to ensure that a dosage form, an active substance, does not move within a very short time from the stomach into the intestinal tract but that it remains in the stomach over a prolonged period of time instead, e.g.:
- Drug products for diagnostic purposes
- The effect of the active substance must be exerted in the stomach (e.g., antacids, antibiotics)
- Improved bioavailability of the active substance in the stomach, duodenum and/or jejunum
- Instability of the active substance in the basic pH range.

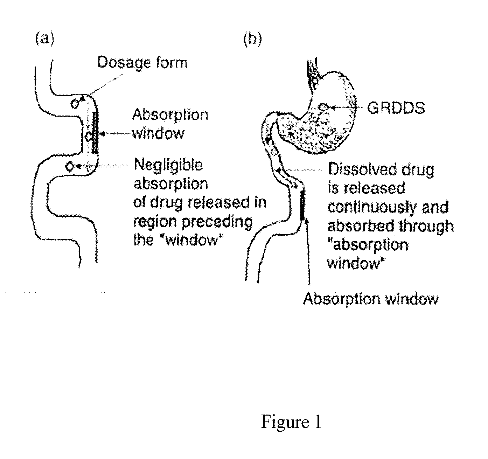

Absorption of the active substance in various dosage forms over time, see Figure 1, showing
(a): Absorption of the active substance in a "conventional" dosage form over time
(b): Absorption of the active substance in a gastroretentive dosage form over time

For tolperisone, a floating tablet based on the hydrodynamic principle and having a lower density than the gastric juice was developed. By adding acid adjuvants, such as citric acid, it is possible to produce a GRDDS (Gastro Retentive Drug Delivery System) that is free from 4-MMPPO.

### 4.2 Examples:

### Example 1(SB080123):

### Preparation from:

| Substance | Fraction planned (%) | Initial weight (g) | Fraction measures (%) |
|---|---|---|---|
| Tolperisone HCl | 50 | 2.50 | 50.00 |
| Citric acid, anhydrous | 10 | 0.50 | 10.00 |
| Methocel K4M | 11.6 | 0.58 | 11.60 |
| Methocel K15M | 11.6 | 0.58 | 11.60 |
| Accurel MP 1000 | 16.6 | 0.83 | 16.60 |

A powdered mixture of 2.5 g of tolperisone hydrochloride and 0.5 g of anhydrous citric acid is produced, and the mixture is pre-compacted. This pre-compacted mixture is mixed with the adjuvants Methocel K4M, Methocel K15M and Accurel MP 1000 and compressed in a tablet press at a pressure higher than 50 kN. A floatable CR tablet is obtained. Methocel K4M and Methocel K15M are water-soluble methylcellulose and hydroxypropyl methylcellulose polymers and are available from Dow Chemical Co., Midland, Michigan, USA. Accurel MP 1000 is a microporous polypropylene powder available from Membrana GmbH / Accurel Systems, Obemburg, Germany.

The analytical assessment confirms a 4-MMPPO content lower than 1.5 ppm, relative to a 41.3% active substance content (tolperisone content).

The tablet core is subjected to coating with Eudragit (Eudragit E or RS or S) film. Eudragit film is made from Eudragit (E or RS or S) polymethacrylates available from Evonik Industries AG, Essen, Germany. The eudragit film is applied via spraying with air pressure.

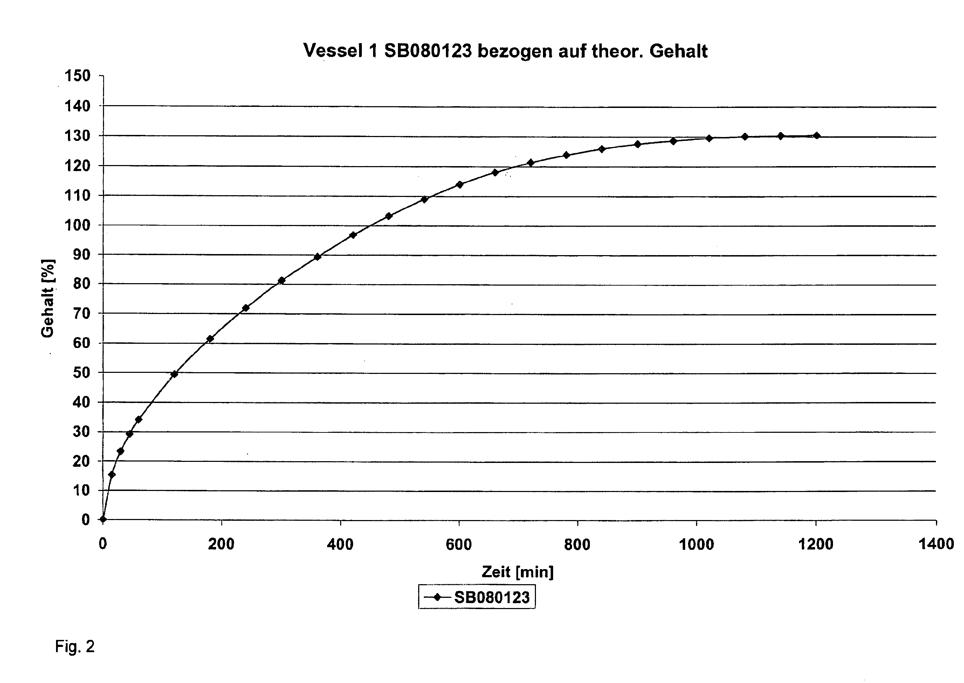

### 4.3 Analytical investigation of release behaviour, see Figure 2

The release curve confirms the extended release:
A Pharm Eu. Method (Basketmethod) for retarded release was used. For this purpose tablets equaling an amount of 300 mg active ingredient "Tolpersione" were put in a basket and dissolved under continuous stirring in 900ml acidic solvent.
Key:
   - 1: Vessel 1 SB080123 (prepared according to Example 1) relative to the theoretical content
   - 2: Assay (%)
   - 3: Time (min)
The curve shows that a complete dissolution of the tablet takes up to more than 800 minutes guaranteeing a retarded release of the active ingredient compared to ordinary IR tablets were a complete release of the active ingredient after 45 minutes is mandatory.

## Claims

1. A GRDDS (Gastro Retentive Drug Delivery System) containing tolperisone and having a 4-MMPPO fraction of less than 1.5 ppm for the extended release of the active substance tolperisone while avoiding the formation of 4-MMPPO in the gastrointestinal tract.
